# EUROPEAN PATENT SPECIFICATION

(11) **EP 3 102 170 B1**
(45) Date of publication and mention of the grant of the patent: **10.10.2018**
(21) Application number: 15703952.0
(22) Date of filing: 03.02.2015
(51) Int. Cl.: A61G 5/12, A61G 7/057, A61G 7/07, A61F 5/37, A63B 23/025

(54) **HEAD SUPPORT APPARATUS**
KOPFSTÜTZVORRICHTUNG
APPAREIL FORMANT APPUI-TÊTE

(30) Priority: 05.02.2014 GB 201401969
(43) Date of publication of application: 14.12.2016
(73) Proprietor: Oakfield Engineering Limited, Maghera, County Londonderry BT46 5SA (GB)
(72) Inventor: O'LOUGHLIN, Joseph, Maghera County Londonderry BT46 5SA (GB)
(74) Representative: FRKelly
(86) International application number: PCT/EP2015/052220
(87) International publication number: WO 2015/117968

(56) References cited:
- US-A- 3 421 500
- US-A- 5 116 359
- US-A1- 2010 292 051

## Description

### FIELD OF THE INVENTION

This invention relates to a head support apparatus for attachment to a wheelchair or bed for preventing the formation of sores between the patient's neck and chin.

### BACKGROUND OF THE INVENTION

Many infirm, paralyzed, or elderly persons have inadequate use or control of their neck muscles. When such persons are seated in a wheelchair, their weak neck muscles often allow their head to fall forwards such that their chin rests upon their chest. This, combined with feeding and drinking difficulties, can lead to the formation of damp and unsanitary conditions between the patient's neck and chin, leading to the formation of sores.

Attempts have been made to provide head supporting strap arrangements that can be worn around the head to maintain the head in an upright position. However, the constant pressure applied to the skin by such strap arrangements can lead to pressure sores. WO 95/01114 discloses a head support for a wheelchair bound patient comprising a headband attached to a frame on the wheelchair via an adjustable cord, whereby the length of the cord can be manually adjusted to progressively lift the patient's head. However, this arrangement is not able to automatically lift and lower the patient's head. It is purely a manually operable head supporting device. US 5116359 discloses a therapeutic exercise device which mechanically assists the human head and neck with intermittent rearward/forward movement.

### SUMMARY OF THE INVENTION

According to the present invention there is provided a head support apparatus for attachment to a wheelchair or bed, the apparatus comprising a headband or harness to be fitted to the patient's head and lifting means attached to the headband or harness for lifting and supporting the patient's head, the lifting means being adapted to periodically raise and support the patient's head and subsequently allow the patient's head to slump forwards, wherein the lifting means is adapted to rotate the head from side to side.
The invention allows the patient's head to be raised and supported to reduce the formation of sores between the patient's neck and chin while avoiding the formation of pressure sores on the head which might otherwise form if the patient's head were supported by the headband or harness for long periods of time. The periodic raising, lowering and rotation of the patient's head may also provide therapeutic excercise for the patient's neck.
The lifting means may comprise a crank arm or wheel, to which the headband or harness may be attached at a location offset from the centre of rotation of the crank arm or wheel.

The crank arm or wheel may be rotated or turned by a motor under the control of a controller. The contoller may be programmed to periodically raise and lower the patient's head at predetermined intervals.
The headband or harness may be attached to the crank arm or wheel via an elongate rope, cord or strap.
The lifting means is preferably mounted within a housing, the housing being adapted to be affixed to a wheelchair or bed. The housing may include a control panel having user controls for controlling the operation of the lifting means.
In a preferred emboidment the lifting means may be adapted to rotate the head from side to side by alternately raising and lower either side of the head or by raising and lowering either side of the head out of phase with one another. Such rotation may provide beneficial excercise for the neck muscles. This may be achieved by connecting separate ropes, cords or straps on each side of the headband or harness, connecting each rope, cord or strap to a separate crank arm or wheel which may be rotated out of phase with one another by a single motor or which may be rotated independently by separate motors.

### BRIEF DESCRIPTION OF THE DRAWINGS

A head support apparatus in accordance with an embodiment of the present invention will now be described, by way of example only, with reference to the accompanying drawings, in which :-
Figure 1 is a side view of a head support apparatus according to an embodiment of the present invention;
Figure 2 is a front view of the head support apparatus of Figure 1;
Figure 3 is a perpsective view of the head support apparatus of Figure 1;
Figure 4 is a detailed view of the head support apparatus of Figure 1; and
Figure 5 is a perspective view of the head support apparatus of Figure 1 with the outer cover removed.

### DESCRIPTION OF THE PREFERRED EMBODIMENT

A illustrated in the drawings, a head support apparatus in accordance with an embodiment of the present invention comprises a housing 2 adapted to be mounted onto a wheelchair or bed within which a patient may be confined.

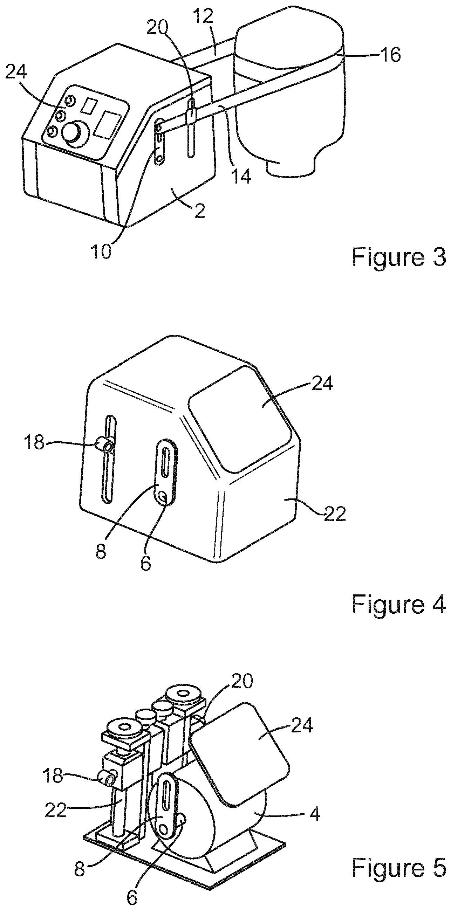

A lifting means is mounted in the housing 2 in the form of a motor (4, Figure 5) drivingly coupled to a drive shaft 6, preferably via suitable gearing, the drive shaft 6 extending transverse to the housing to extend through apertures on either side of the housing. A pair of parallel crank arms 8,10 are mounted on either end of the drive shaft to be located on either side of the housing 2.

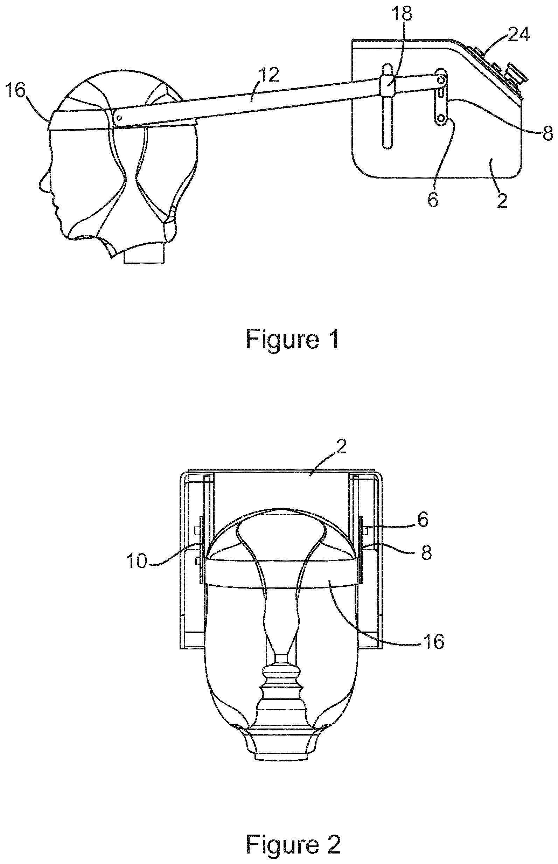

Each crank arm 8,10 is coupled to a strap 12,14 attached to a respective side of a harness or headband 16 to be fitted around the head of a patient, as shown in Figures 2 to 3. The straps 12,14 are mounted on either side of the harness or headband 16 such that the patient's head is supported in both a vertical and a horizontal plane. A slot is provided in each crank arm 8,10 such that the distance between the point and which the respective strap 12,14 is coupled to the crank arm 8,10 and the rotational axis of the crank arm 8,10 can be adjusted to adjust the stroke or range of motion of the patient's head between raised and lowered positions. It is envisaged that each crank arm 8,10 may be replaced by respective discs to which the straps 12,14 may be respectively attached at locations spaced from the centre of rotation of each disc.

The straps 12,14 pass through respective guides 18,20 provided on either side of the housing 2. As shown in Figure 5, the height of the guides 18,20 may be adjusted to suit different sized patients. In the embodiment shown, the guides 18,20 are mounted on threaded support posts 22.

A programmable controller, incorporating a timer, is associated with the motor for periodically operating the motor at predetermined intervals, such that the motor rotates the crank arms, raising and lowering the head of the patient.

A control panel 24 is provided on a rear side of the housing enabling a user to control the operation of the controller and thus the lifting means.

In use, the harness or headband 16 is attached to the patient and the control panel 24 is used to programme the controller to enable the lifting means to periodically lift and support the patient's head, holding the patient's chin away from the patient's neck, and periodically lower the patient's head, releiveing pressure from the harness or headband.

Each strap 12,14 may be connected to a respective crank arm 8,10 such the straps 12,14 pull against the harness or headband 16 out of phase with one another, for example by mounting the cranks 8,10 out of phase with one another on the drive shaft 6, preferably 180° out of phase with one another, such that operation of the motor 4 causes the head to rotate from side to side. Such movement may provide beneficial excercise for the neck. Alternatively each strap 12,14 may be connected to a respective crank arm 8,10 driven by a respective and separate motor so that the two cranks arms 8,10 can be rotated independently under the control of the controller.
The invention is not limited to the embodiment(s) described herein but can be amended or modified within the scope of the present invention as defined by the claims .

## Claims

1. A head support apparatus for attachment to a wheelchair or bed, the apparatus comprising a headband or harness (16) to be fitted to the patient's head and lifting means attached to the headband or harness for lifting and supporting the patient's head, the lifting means being adapted to periodically raise and support the patient's head and subsequently allow the patient's head to slump forwards, **characterised in that** the lifting means is adapted to rotate the head from side to side.

2. A head support apparatus as claimed in claim 1, wherein the lifting means comprises a crank arm or wheel (8,10), to which the headband or harness (16) is attached at a location offset from the centre of rotation of the crank arm or wheel.

3. A head support apparatus as claimed in claim 2, wherein the crank arm or wheel (8,10) is rotated or turned by a motor under the control of a controller.

4. A head support apparatus as claimed in claim 3, wherein the contoller is programmed to periodically raise, lower and rotate the patient's head at predetermined intervals.

5. A head support apparatus as claimed in any of claims 2 to 4, wherein the headband or harness (16) is attached to the crank arm or wheel (8,10) via an elongate rope, cord or strap (12,14).

6. A head support apparatus as claimed in any preceding claim, wherein the lifting means is mounted within a housing (2), the housing being adapted to be affixed to a wheelchair or bed.

7. A head support apparatus as claimed in claim 6, wherein the housing (2) includes a control panel (24) having user controls for controlling the operation of the lifting means.

8. A head support apparatus as claimed in any preceding claim, wherein the lifting means is adapted to alternately or indepedently raise and lower either side of the head.

9. A head support apparatus as claimed in claim 8, wherein the lifting means is adapted to raise and lower either side of the head out of phase with one another.

10. A head support apparatus as claimed in any preceding claim, comprising a housing (2), said lifting means comprising pair of crank arms (8,10) mounted on either side of the housing (2), each crank arm (8,10) being coupled to a respective strap (12,14) attached to a respective side of the harness or headband (16) such that the patient's head is supported in both a vertical and a horizontal plane, wherein the crank arms (8,10) are rotatably driven to raise, lower and rotate the patient's head.

11. A head support apparatus as claimed in claim 10, wherein the crank arms (8,10) are mounted on a common drive shaft extending through the housing.

12. A head support apparatus as claimed in claim 11, wherein the crank arms (8,10) are angularly offset from one another such that rotation of the crank arms causes either side of the patient's head to be raised and lowered out of phase with one another, thus rotating the head.

13. A head support apparatus as claimed in claim 10, wherein each crank arm (8,10) is coupled to a separate drive means such that the cranks arms can be rotated indepenedently from one another under the control of a common controller.

14. A head support apparatus as claimed in any of claims 10 to 13, wherein a slot is provided in each crank arm (8,10) such that the distance between the point and which the respective strap (12,14) is coupled to the crank arm (8,10) and the rotational axis of the crank arm can be adjusted to adjust the stroke or range of motion of the patient's head between raised and lowered positions

15. A head support apparatus as claimed in any of claims 10 to 14 wherein the straps pass through respective guides provided on either side of the housing, wherein the position of each guide on the housing is vertically adjustable to suit different sized patients.

## Patentansprüche

1. Kopfstützvorrichtung zum Anbringen an einem Rollstuhl oder einem Bett, wobei die Vorrichtung ein am Kopf des Patienten anzulegendes Stirnband oder Geschirr (16) und ein an dem Stirnband oder Geschirr angebrachtes Hebemittel zum Heben und Stützen des Kopfs des Patienten umfasst, wobei das Hebemittel dazu angepasst ist, den Kopf des Patienten regelmäßig anzuheben und zu stützen und anschließend den Kopf des Patienten nach vorne fallen zu lassen, **dadurch gekennzeichnet, dass** das Hebemittel dazu angepasst ist, den Kopf von Seite zu Seite zu drehen.

2. Kopfstützvorrichtung nach Anspruch 1, wobei das Hebemittel einen Kurbelarm oder ein Kurbelrad (8, 10) umfasst, an dem das Stirnband oder Geschirr (16) an einem vom Drehmittelpunkt des Kurbelarms oder Kurbelrads versetzten Ort angebracht ist.

3. Kopfstützvorrichtung nach Anspruch 2, wobei der Kurbelarm oder das Kurbelrad (8, 10) von einem Motor unter der Steuerung eines Kontrollers gedreht oder rotiert wird.

4. Kopfstützvorrichtung nach Anspruch 3, wobei der Kontroller dazu programmiert ist, den Kopf des Patienten in vorbestimmten Zeitabständen regelmäßig anzuheben, abzusenken und zu drehen.

5. Kopfstützvorrichtung nach einem der Ansprüche 2 bis 4, wobei das Stirnband oder Geschirr (16) mittels eines langgestreckten Seils, einer langgestreckten Schnur oder eines langgestreckten Riemens (12, 14) an dem Kurbelarm oder Kurbelrad (8, 10) angebracht ist.

6. Kopfstützvorrichtung nach einem der vorangehenden Ansprüche, wobei das Hebemittel in einem Gehäuse (2) montiert ist, wobei das Gehäuse dazu angepasst ist, an einem Rollstuhl oder Bett befestigt zu werden.

7. Kopfstützvorrichtung nach Anspruch 6, wobei das Gehäuse (2) ein Bedienfeld (24) umfasst, das Benutzerbedienungselemente zum Steuern des Betriebs des Hebemittels aufweist.

8. Kopfstützvorrichtung nach einem der vorangehenden Ansprüche, wobei das Hebemittel dazu angepasst ist, beide Seiten des Kopfs abwechselnd oder unabhängig anzuheben und abzusenken.

9. Kopfstützvorrichtung nach Anspruch 8, wobei das Hebemittel dazu angepasst ist, beide Seiten des Kopfs gegenphasig anzuheben und abzusenken.

10. Kopfstützvorrichtung nach einem der vorangehenden Ansprüche, umfassend ein Gehäuse (2), wobei das Hebemittel ein Paar auf beiden Seiten des Gehäuses (2) montierte Kurbelarme (8, 10) umfasst, wobei die Kurbelarme (8, 10) jeweils an einen jeweiligen Riemen (12, 14) gekoppelt sind, der an einer jeweiligen Seite des Geschirrs oder Stirnbands (16) angebracht ist, sodass der Kopf des Patienten sowohl in einer vertikalen als auch einer horizontalen Ebene gestützt wird, wobei die Kurbelarme (8, 10) drehbar angetrieben werden, um den Kopf des Patienten anzuheben, abzusenken und zu drehen.

11. Kopfstützvorrichtung nach Anspruch 10, wobei die Kurbelarme (8, 10) auf einer gemeinsamen Antriebswelle montiert sind, die sich durch das Gehäuse erstreckt.

12. Kopfstützvorrichtung nach Anspruch 11, wobei die Kurbelarme (8, 10) voneinander winkelversetzt sind, sodass das Drehen des Kurbelarms bewirkt, dass beide Seiten des Kopfs des Patienten gegenphasig angehoben und abgesenkt werden, sodass der Kopf gedreht wird.

13. Kopfstützvorrichtung nach Anspruch 10, wobei die Kurbelarme (8, 10) jeweils an ein getrenntes Antriebsmittel gekoppelt sind, sodass die Kurbelarme unter der Steuerung eines gemeinsamen Kontrollers unabhängig voneinander gedreht werden können.

14. Kopfstützvorrichtung nach einem der Ansprüche 10 bis 13, wobei in jedem Kurbelarm (8, 10) ein Schlitz bereitgestellt ist, sodass der Abstand zwischen der Stelle, an der der jeweilige Riemen (12, 14) an den Kurbelarm (8, 10) gekoppelt ist, und der Drehachse des Kurbelarms verstellt werden kann, um den Hub oder Bewegungsbereich des Kopfs des Patienten zwischen der angehobenen und der abgesenkten Stellung zu verstellen.

15. Kopfstützvorrichtung nach einem der Ansprüche 10 bis 14, wobei die Riemen durch jeweilige, auf beiden Seiten des Gehäuses bereitgestellte Führungen laufen, wobei die Lage der Führungen an dem Gehäuse jeweils vertikal, passend zu Patienten unterschiedlicher Größe verstellbar ist.

## Revendications

1. Appareil de soutien pour la tête servant à des fins de fixation sur un fauteuil roulant ou un lit, l'appareil comportant un serre-tête ou harnais (16) destiné à être placé sur la tête d'un patient et un moyen de levage attaché sur le serre-tête ou harnais à des fins de levage et de soutien de la tête du patient, le moyen de levage étant adapté pour relever et soutenir périodiquement la tête du patient et par la suite permettre à la tête du patient de tomber en avant, **caractérisé en ce que** le moyen de levage est adapté pour faire tourner la tête d'un côté et de l'autre.

2. Appareil de soutien pour la tête selon la revendication 1, dans lequel le moyen de levage comporte un bras de manivelle ou vilebrequin (8, 10) auquel le serre-tête ou harnais (16) est attaché au niveau d'un emplacement décalé par rapport au centre de rotation du bras de manivelle ou vilebrequin.

3. Appareil de soutien pour la tête selon la revendication 2, dans lequel le bras de manivelle ou vilebrequin (8, 10) est mis en rotation ou tourné par un moteur sous la commande d'un dispositif de commande.

4. Appareil de soutien pour la tête selon la revendication 3, dans lequel le dispositif de commande est programmé pour, périodiquement, relever, abaisser et faire tourner la tête du patient selon des intervalles prédéterminés.

5. Appareil de soutien pour la tête selon l'une quelconque des revendications 2 à 4, dans lequel le serre-tête ou harnais (16) est attaché au bras de manivelle ou vilebrequin (8, 10) par le biais d'une corde, cordelette ou sangle allongée (12, 14).

6. Appareil de soutien pour la tête selon l'une quelconque des revendications précédentes, dans lequel le moyen de levage est monté à l'intérieur d'un boîtier (2), le boîtier étant adapté pour être fixé sur un fauteuil roulant ou un lit.

7. Appareil de soutien pour la tête selon la revendication 6, dans lequel le boîtier (2) comprend un panneau de commande (24) ayant des commandes d'utilisateur servant à commander le fonctionnement du moyen de levage.

8. Appareil de soutien pour la tête selon l'une quelconque des revendications précédentes, dans lequel le moyen de levage est adapté pour relever et abaisser de manière alternée ou de manière indépendante chaque côté de la tête.

9. Appareil de soutien pour la tête selon la revendication 8, dans lequel le moyen de levage est adapté pour relever et abaisser chaque côté de la tête de manière déphasée l'un par rapport à l'autre.

10. Appareil de soutien pour la tête selon l'une quelconque des revendications précédentes, comportant un boîtier (2), ledit moyen de levage comportant une paire de bras de manivelle (8, 10) montés de part et d'autre du boîtier (2), chaque bras de manivelle (8, 10) étant accouplé à une sangle respective (12, 14) attachée sur un côté respectif du harnais ou serre-tête (16) de telle sorte que la tête du patient est soutenue à la fois dans un plan vertical et un plan horizontal, dans lequel les bras de manivelle (8, 10) sont entraînés de manière rotative pour soulever, abaisser et faire tourner la tête du patient.

11. Appareil de soutien pour la tête selon la revendication 10, dans lequel les bras de manivelle (8, 10) sont montés sur un arbre d'entraînement commun s'étendant au travers du boîtier.

12. Appareil de soutien pour la tête selon la revendication 11, dans lequel les bras de manivelle (8, 10) sont décalés de manière angulaire l'un par rapport à l'autre de telle sorte que la rotation des bras de manivelle entraîne chaque côté de la tête du patient à être soulevé et abaissé de manière déphasée l'un par rapport à l'autre, pour ainsi faire tourner la tête.

13. Appareil de soutien pour la tête selon la revendication 10, dans lequel chaque bras de manivelle (8, 10) est accouplé à un moyen d'entraînement séparé de telle sorte que les bras de manivelle peuvent être tournés de manière indépendante l'un par rapport à l'autre sous la commande d'un dispositif de commande commun.

14. Appareil de soutien pour la tête selon l'une quelconque des revendications 10 à 13, dans lequel une fente est mise en oeuvre dans chaque bras de manivelle (8, 10) de telle sorte que la distance entre le point au niveau duquel la sangle respective (12, 14) est accouplée au bras de manivelle (8, 10) et l'axe de rotation du bras de manivelle peut être ajustée pour ajuster la course ou l'étendue de mouvement de la tête du patient entre les positions relevée et abaissée.

15. Appareil de soutien pour la tête selon l'une quelconque des revendications 10 à 14, dans lequel les sangles passent au travers de dispositifs de guidage respectifs mis en oeuvre de part et d'autre du boîtier, dans lequel la position de chaque dispositif de guidage sur le boîtier est ajustable dans le sens vertical à des fins d'adaptation à des patients de différentes tailles.
